# EUROPÄISCHE PATENTSCHRIFT

(11) **EP 3 111 220 B1**
(45) Veröffentlichungstag und Bekanntmachung des Hinweises auf die Patenterteilung: **25.12.2019**
(21) Anmeldenummer: 15707904.7
(22) Anmeldetag: 26.02.2015
(51) Int. Cl.: C12Q 1/6806, G01N 33/569, C12N 15/10

(54) **VERFAHREN FÜR DIE MOLEKULARDIAGNOSTIK ZUM ANREICHERN EINER NUKLEINSÄURE AUS EINER BIOLOGISCHEN PROBE**
PROCEDURES FOR MOLECULAR DIAGNOSTICS FOR ENRICHING A NUCLEIC ACID FROM A BIOLOGICAL SAMPLE
PROCÉDURES POUR DIAGNOSTICS MOLÉCULAIRES POUR ENRICHIR UN ACIDE NUCLÉIQUE D'UN ÉCHANTILLON BIOLOGIQUE

(30) Priorität: 26.02.2014 EP 14156810; 03.04.2014 DE 102014206441
(43) Veröffentlichungstag der Anmeldung: 04.01.2017
(73) Patentinhaber: EarlyBio GmbH, 13465 Berlin (DE)
(72) Erfinder: HAYDEN, Oliver, 91074 Herzogenaurach (DE); WARNAT, Gerald, 21702 Ahlerstedt (DE); HELOU, Michael Johannes, 93051 Regensburg (DE); REISBECK, Mathias, 93055 Regensburg (DE); RICHTER, Lukas, 96114 Hirschaid (DE)
(74) Vertreter: DTS Patent- und Rechtsanwälte Schnekenbühl und Partner mbB
(86) Internationale Anmeldenummer: PCT/EP2015/053971
(87) Internationale Veröffentlichungsnummer: WO 2015/128396

(56) Entgegenhaltungen:
- EP-A2- 0 311 163
- WO-A1-2008/101192
- WO-A1-2009/092068
- WO-A2-2012/012725
- DE-A1-102005 009 479
- DE-A1-102009 047 801
- DE-A1-102011 004 806
- DE-A1-102011 077 905
- DE-A1-102012 210 598
- D. D. DANIKAS ET AL: "Prognostic value of phagocytic activity of neutrophils and monocytes in sepsis. Correlation to CD64 and CD14 antigen expression", CLINICAL & EXPERIMENTAL IMMUNOLOGY, Bd. 154, Nr. 1, 1. Oktober 2008 (2008-10-01), Seiten 87-97, XP055191026, ISSN: 0009-9104, DOI: 10.1111/j.1365-2249.2008.03737.x
- KANG-YI LIEN ET AL: "Extraction of genomic DNA and detection of single nucleotide polymorphism genotyping utilizing an integrated magnetic bead-based microfluidic platform", MICROFLUIDICS AND NANOFLUIDICS, SPRINGER, BERLIN, DE, Bd. 6, Nr. 4, 6. August 2008 (2008-08-06), Seiten 539-555, XP019667803, ISSN: 1613-4990
- MICHAEL HELOU ET AL: "Time-of-flight magnetic flow cytometry in whole blood with integrated sample preparation", LAB ON A CHIP, Bd. 13, Nr. 6, 1. Januar 2013 (2013-01-01) , Seite 1035, XP055074753, ISSN: 1473-0197, DOI: 10.1039/c3lc41310a

## Beschreibung

Die Erfindung betrifft ein Verfahren für die Molekulardiagnostik zum Anreichern einer Nukleinsäure aus einer biologischen Probe.

In der Molekulardiagnostik werden bisher vor allem zwei Methoden für eine Nukleinsäurediagnostik genutzt. Einerseits kann mit dem Produkt Septifast von Roche eine Nukleinsäure direkt aus zumindest 1,5 Milliliter Vollblut mittels eines initialen Zellaufschlusses, also einer initialen Zelllyse, eine Nukleinsäureextraktion durchgeführt werden. Zum anderen kann mittels eines Produktes von Molzym eine Nukleinsäure über eine selektive Lyse extrahiert werden. Dort findet zunnächst eine milde Lyse gefolgt von einer Zentrifugation statt. Darauf folgen eine harte Lyse und ein Aufreinigen der Nukleinsäure.

Für eine besonders schnelle Diagnostik, insbesondere eine patientennahe Labordiagnostik, die auch von gewöhnlichem medizinischem Personal ohne Laborausbildung durchgeführt werden kann, gibt es gegenwärtig kein geeignetes Verfahren. Dies gilt insbesondere für eine schnelle Sepsisdiagnostik, mit der eine Sepsis von einem systemischen inflammatorischen Responssyndrom (SIRS) differenziert, beziehungsweise in einem Frühstadium bei operierten Patienten erkannt werden kann, um darauffolgend auch Bakterien oder Resistenzen zu diagnostizieren. In einem Frühstadium mit wenigen Bakterien haben die bekannten Verfahren zudem große Schwierigkeiten, verlässliche Ergebnisse zu erzielen. Gerade bei Sepsispatienten wäre jedoch eine frühe Identifizierung und Resistenztestung hoch relevant, da pro Stunde die Mortalität von Sepsispatienten um durchschnittlich 7 Prozent ohne frühzeitige Diagnostik steigt. Bisher sind jedoch zeitaufwändig mehrere unabhängige diagnostische beziehungsweise präanalytische Verfahren notwendig, um nach einem Sepsisverdacht eine wirksame Therapie starten zu können.

Die WO2008/101192 A1 offenbart ein Verfahren, um selektiv eine Nukleinsäure eines ersten Zelltyps aus einer forensischen Probe mit Zellen des ersten Zelltyps und zumindest auch eines zweiten Zelltyps sowie weiteren Verunreinigungen zu isolieren. Die Zellen können dabei mit magnetischen Partikeln markiert werden und dadurch durch ein magnetisches Feld vom Rest der Probe abgetrennt werden. Infolge werden die Verunreinigungen aus der Probe entfernt und die besagten Zellen behalten. Nach einem selektiven Lysieren der Zellen des ersten Typs und einem entsprechenden Isolieren der Nukleinsäuren können beispielsweise auch die Nukleinsäuren der zweiten Zellen isoliert werden. Bei den ersten oder zweiten Zellen kann es sich dabei auch um Neutrophile, Monozyten oder Makrophagen handeln.

Die DE 102012210598 A1 offenbart eine Anordnung zur Quantifizierung von Zellen unter Unterscheidung wenigstens zweier verschiedener Größen von Zell-Arten und/oder Zellkonglomerat-Arten in einer Zellsuspension, mit einem magnetfeldsensitiven Sensor.

Die DE 102011004806 A1 offenbart eine Vorrichtung zur magnetischen Durchflusszytometrie, bei welcher markierte Zellen auf einem Halbleiterchip angereichert werden können. Insbesondere gewährleistet diese Vorrichtung eine lange Anreicherungsstrecke für hohen Durchsatz von großen Probenvolumina.

Die WO 2009/092068 A1 offenbart die differenzierte Analyse von Blutphagozyten, die z. B. Tumorzellen oder Pathogene phagozytiert haben im Vergleich von Blutphagozyten, die keine Pathogene oder Tumorzellen phagozytiert haben. Nach bestimmten Ausführungsformen enthalten Phagozyten Zellen und/oder Fragmente und/oder Bestandteile davon, die im Blut zirkulieren und für eine bestimmte Krankheit oder einen bestimmten Zustand charakteristisch sind. Der Inhalt der Phagozyten liefert ein Markerprofil für die o.g. Krankheit oder den o.g. Zustand. Der Vergleich der DNA-Expressionsprofile oder Proteinexpressionsprofile von phagozytären und nicht-phagozytären weißen Blutzellen führt zum Nachweis von tumorspezifischen, krankheitsspezifischen oder konditionsspezifischen DNA-Signaturen in Phagozyten, die in der nicht-phagozytären Zelle entweder nicht exprimiert oder unterexprimiert wurden.

Die WO 2012/012725 A2 offenbart Verfahren, um Krankheiten oder Zustände mit Hilfe von Phagozyten mit unterschiedlichen DNA-Gehalten (d.h. >2n und =2n) nachzuweisen. In einigen Ausführungsformen werden zwei Teilpopulationen von Phagozyten verwendet, wobei die Phagozyten mit einem DNA-Gehalt größer als 2n (">2n phagozytische Zellen") als Surrogate für erkrankte Zellen dienen, während die phagozytischen Zellen mit einem DNA-Gehalt von 2n ("=2n phagozytische Zellen") als Kontrollzellen dienen.

Danikas et al. offenbart die Ausbildung von Antigenen bei einer Sepsis. Insbesondere wird die Expression von CD64 und CD14 auf Monozyten und polymorphnukleären Zellen (PMN) mit dem Patientenüberleben und der Aktivität der Phagozyten korreliert. Patienten mit einer relativ hohen Expression von CD64 und einer relativ hohen Aktivität von Monozyten und PMNs zeigten ein schlechteres Outcome (Danikas et al., "Prognostic value of phagocytic activity of neutrophils and monocytes in sepsis. Correlation to CD64 and CD14 antigen expression", Clin Exp Immunol. 2008 Oct 154(1):87-97).

Eine der vorliegenden Erfindung zu Grunde liegende Aufgabe ist es, ein Verfahren für die Molekulardiagnostik zum Anreichern von Nukleinsäure aus einer biologischen Probe bereitzustellen, welches besonders schnell und einfach durchzuführen ist, so dass es als Routinediagnostik einer patientennahen Labordiagnostik von Personal ohne Laborausbildung durchgeführt werden kann.

Diese Aufgabe wird von dem Verfahren gemäß dem unabhängigen Patentanspruch gelöst. Weitere vorteilhafte Ausführungsformen ergeben sich aus den abhängigen Patentansprüchen, der Beschreibung und den Figuren.

Die Erfindung betrifft ein Verfahren für die Molekulardiagnostik, zum Anreichern von Nukleinsäuren phagozytierter Zellen eines ersten Zelltyps aus einer zuvor entnommenen biologischen Probe eines Patienten, wobei die biologische Probe Zellen eines weiteren, phagozytierenden Zelltyps, die jeweils eine Zelle des ersten Zelltyps phagozytieren, umfasst. Beispielsweise kann es sich bei den Nukleinsäuren um eine Vielzahl von Exemplaren mit einer bestimmten und/oder identischen Nukleinsäurestruktur handeln. Es können damit dann beispielsweise Nukleinsäuren aus z.B. von pathogenen Zellen, die beispielsweise von polymorphnukleären Zellen des immunsystems phagozytiert wurden, angereichert werden. Bei der biologischen Probe kann es sich insbesondere um eine unlysierte biologische Probe handeln, mit der nach einer Entnahme aus dem Patienten noch kein Lyse durchgeführt wurde. Insbesondere kann es sich hier auch um eine chemisch unbehandelte biologische Probe handeln. Damit das Verfahren einfach und schnell durchgeführt werden kann, erfolgt zunächst ein Markieren von zumindest einem Anteil der phagozytierenden Zellen der biologischen Probe durch für die phagozytierenden Zellen spezifische Antikörper, wobei die Antikörper jeweils an einen magnetischen Marker gekoppelt sind. Da nur zumindest ein Anteil der phagozytierenden Zellen markiert ist, können auch, beispielsweise aus statistischen Gründen, in der biologischen Probe nach dem Markieren noch unmarkierte phagozytierende Zellen vorliegen. Zu diesem Zeitpunkt ist noch keine Lyse der phagozytierenden Zellen durch einen initialen Zellaufschluss erfolgt, es handelt sich also insbesondere um eine unlysierte biologische Probe. Die Antikörper sind spezifisch für Zellmembranproteine eines vorbestimmten Zellmembranproteintyps, welche von den phagozytierenden Zellen nur im Falle einer erfolgten Phagozytose exprimiert werden, also insbesondere spezifisch für Zellmembranproteine vom Typ eines Sepsisbiomarker wie beispielsweise CD64, vgl. hierzu Clin Experim Immunol, 154: 87-97 (2008). Auf das Markieren folgt ein Abtrennen, also ein Separieren, der markierten phagozytierenden Zellen aus der biologischen Probe mittels eines durch eine Zelltrennvorrichtung, beispielsweise durch ein Durchflusszytometer, angelegten Magnetfeldes, wodurch ein Anreichern der markierten phagozytierenden Zellen und damit ein Anreichern der Nukleinsäuren der phagozytierten Zellen erfolgt. Das Verfahren ist also insbesondere als Präanalyseverfahren zu bezeichnen. Die Nukleinsäuren können insbesondere einzel-oder doppelsträngige Desoxyribonukleinsäuren (DNA) oder Ribonukleinsäuren der phagozytierten Zellen umfassen.

Es wird hier eine sehr frische biologische Probe verwendet, die einem Patienten entnommen wurde. Das hat den Vorteil, dass die biologische Probe den Zustand des biologischen Trägers gut repräsentiert und z.B. eine Infektion sehr schnell erkannt werden kann und die phagozytierten Zellen mit hoher Wahrscheinlichkeit noch nicht verdaut sind, vgl. hierzu J. Biol. Chem. 2002, 277:30463- 30468.

Das Verfahren hat den Vorteil, dass das Anreichern ohne eine initiale, also vorherige, aufwendige Lyse erfolgt, so dass eine erhebliche Zeitersparnis eintritt. Wichtig ist, dass hierbei die Nukleinsäuren der ersten Zellen zunächst intakt bleiben, da die phagozytierten Zellen zwar schnell phagozytiert werden, aber der darauffolgende Verdauungsprozess durch die phagozytierenden Zellen sehr langsam abläuft. Typischerweise ist die Phagozytose einer Zelle durch eine phagozytierende Zelle in Sekunden bis Minuten nach einem Kontakt der Zellen abgeschlossen, die anschließende Verdauung erfolgt jedoch in einem Zeitraum von einer oder mehreren Stunden, vgl. hierzu J. Biol. Chem. 2002, 277:30463-30468. Des Weiteren liegt durch das Abtrennen der markierten Zellen von der Probe für nachfolgende weitere Verfahrensschritte ein im Vergleich zu herkömmlichen Methoden reduzierter störender Hintergrund vor, da vor allem Hämoglobin, freie DNA und DNA von phagozytierenden Zellen der biologischen Probe frühzeitig von den die Nukleinsäuren enthaltenden Teilen der biologischen Probe, also den markierten phagozytierenden Zellen mit den in diesen enthaltenen phagozytierten Zellen, abgetrennt werden. Zudem ermöglicht die Verwendung der magnetischen Marker eine Kombination des Anreicherungsprozesses mit einem qualitativen Nachweis oder einem Quantifizierungsschritt für die phagozytierenden Zellen. Dies ermöglicht eine arbeits-und zeitsparende Mehrfachverwendung der Marker. Dementsprechend kann das erfindungsgemäße Verfahren, vorzugsweise zwischen dem Markieren und dem Separieren, ein Detektieren und/oder Quantifizieren der markierten Zellen, insbesondere durch Bestimmen einer Zellkonzentration, umfassen.

In einer besonders vorteilhaften Ausführungsform ist vorgesehen, dass die Antikörper jeweils an zumindest einen paramagnetischen Marker, insbesondere an zumindest einen superparamagnetischen Marker, gekoppelt sind und/oder vor dem Abtrennen ein Erfassen der markierten phagozytierenden Zellen durch ein magnetisches Durchflusszytometer erfolgt. Dabei umfasst das Erfassen insbesondere ein Quantifizieren der phagozytierenden Zellen, welches wiederum insbesondere ein Bestimmen oder Ermitteln einer Anzahl der markierten phagozytierenden Zellen, insbesondere pro durch das Durchflusszytometer strömende Probevolumen, und/oder ein Bestimmen eines Durchmessers und/oder eines Volumens und/oder einer Fließgeschwindigkeit der jeweiligen markierten phagozytierenden Zelle, insbesondere einer Durchmesserverteilung der markierten phagozytierenden Zellen umfasst. Das hat den Vorteil, dass so durch die Mehrfachverwendung der Marker ein Kombinieren von dem Anreicherungs- und dem Nachweisverfahren erfolgt, so dass hier erneut Zeit und zusätzliche Arbeitsschritte, welche gegebenenfalls eine Schulung erfordern, eingespart werden.

Durch das Quantifizieren ist zum Beispiel über die erfassten Zelldurchmesser auch ein spezifisches Detektieren möglich, ob die phagozytierenden Zellen überhaupt phagozytiert haben, und im Falle eine Phagozytose, in welchem Umfang. Über beispielsweise eine einfache Volumenmessung mit einer magnetischen Laufzeitmessung kann hier beispielsweise eine Konzentration der markierten Zellen bestimmt werden. Dies kann sehr schnell erfolgen und innerhalb weniger Minuten abgeschlossen sein. Ergebnis ist eine sehr reine, definierte Probe mit bekannten Eigenschaften, anhand derer bereits überprüft werden kann, ob diese Probe für weitere und gegebenenfalls zeitaufwendige und teure Diagnostikverfahren geeignet ist.

Unter einer definierten Probe ist hier und im Folgenden eine Probe zu verstehen, deren Zusammensetzung bekannt ist. Insbesondere ist also bekannt, welche Zelltypen und/oder sonstige Bestandteile in welchen Anteilen und/oder in welcher Anzahl in der Probe vorhanden sind, und mit welcher Genauigkeit diese Informationen aufweisen.

In einer weiteren Ausführungsform ist vorgesehen, dass das Abtrennen der markierten phagozytierenden Zellen von einem Volumenstrom der biologischen Probe in einer Zellsortiereinrichtung mittels des angelegten Magnetfeldes in einem Sortiertopf der Zellsortiereinrichtung erfolgt und ein Waschen oder Reinigen der abgetrennten phagozytierenden Zellen in dem Sortiertopf mittels einer durch die Zellsortiereinrichtung strömenden Pufferlösung auf das Abtrennen folgt, insbesondere unter Vorliegen des angelegten Magnetfeldes. Das hat den Vorteil, dass ein besonders effizientes Entfernen der nicht markierten Probenkomponenten, wie beispielsweise im Falle einer Vollblutprobe, Erythrozyten, freies Hämoglobin, nicht markierte Leukozyten oder sonstige Bestandteile des Blutes, erfolgt. Die gereinigten abgetrennten phagozytierenden Zellen können dann aus dem Sortiertopf auf einfache Art und Weise entnommen werden. Zudem wird in diesem Fall im Vergleich zu bekannten Aufreinigungsmethoden ein effizientes oder nahezu vollständiges Reduzieren störender, für das Anreichern der Nukleinsäuren unbenötigter Komponenten der biologischen Probe erzielt. Diese unbenötigten Komponenten bilden sonst insbesondere für ein später gegebenenfalls erfolgendes Amplifizieren der Nukleinsäuren mittels beispielsweise einer Polymerase-Kettenreaktion einen störenden Hintergrund. Auch wird so eine sehr genau definierte Probe erzeugt, die hochkonzentriert ist und von der bereits wenige Mikroliter für eine weitere Diagnostik ausreichend sind.

Bei der Zellsortiereinrichtung kann es sich beispielsweise um eine Kartusche oder ein Cartridge handeln, in welche die jeweilige Probe eingebracht, insbesondere eingespritzt, werden kann. Die Kartusche kann eine auswechselbare Kartusche für eine Haltevorrichtung, insbesondere einen Durchflusszytometer, sein, in welcher das Magnetfeld mit einer vorbestimmten Ausrichtung und Stärke auf die in der Kartusche enthaltenen, und insbesondere durch die Kartusche strömenden, jeweilige Probe wirken kann.

In einer vorteilhaften Ausführungsform ist vorgesehen, dass ein selektives Lysieren eines vorbestimmten Anteils der abgetrennten phagozytierenden Zellen stattfindet, sowie danach ein weiteres Markieren der von den lysierten Zellen zuvor phagozytierten Zellen mit einem magnetischen, insbesondere paramagnetischen, Marker. Durch das selektive Lysieren werden also nur phagozytierende Zellen, nicht aber die phagozytierten Zellen lysiert. Der vorbestimmte Anteil kann beispielsweise aus den zuvor abgetrennten phagozytierenden Zellen herauspipettiert werden. Darauf folgt ein weiteres Abtrennen der phagozytierten Zellen in einem weiteren Zellsortierer oder Flusssortierer in einen Sortiertopf des weiteren Zellsortierers mittels eines weiteren oder des bereits in vorherigen Verfahrensschritten gegebenenfalls angelegten Magnetfeldes. Bei dem magnetischen Marker kann es sich auch um einen superparamagnetischen Marker handeln. Das hat den Vorteil, dass der Verdau der phagozytierten Zellen durch die phagozytierenden Zellen gestoppt wird und die Nukleinsäuren von der Membran der phagozytierten Zellen geschützt erhalten bleiben. Auch können sich die phagozytierten Zellen so gege-benenfalls zumindest teilweise wieder vermehren, was ihren Nachweis erleichtert. Das weitere Markieren durch magnetische Marker ermöglicht analog zu dem vorherigen Trennen und Quantifizieren der phagozytierenden Zellen ein kombiniertes Trennen und Quantifizieren der phagozytierten Zellen. Durch den sich wiederholenden Ablauf mit jeweils der biologischen Probe und nun einem Anteil der phagozytierenden, dann lysierten Zellen, wird der gesamte Arbeitsablauf weiter vereinfacht. Dies hat erneut eine Zeitersparnis und eine geringere fachliche Anforderung an das Bedienpersonal zur Folge.

Hierbei kann insbesondere ein Waschen der phagozytierten Zellen in dem Sortiertopf mittels einer durch den Zellsortierer strömenden Pufferlösung auf das weitere Abtrennen folgen, insbesondere unter Vorliegen des angelegten Magnetfeldes. Das hat den Vorteil, dass die nicht durch Verdau aufgeschlossenen phagozytierten Zellen von unerwünschten Nukleinsäurekomponenten und sonstigen Fragmenten der phagozytierenden Zellen gereinigt wird. Dies ergibt eine sehr reine und angereicherte Probe von Zellen des ersten Zelltyps, wie sie beispielsweise für neuere und teure Next Generation Sequencing (NGS)-Technologien erforderlich ist.

Es kann auch vorgesehen sein, dass ein Erfassen, insbesondere ein Quantifizieren, der phagozytierten Zellen des ersten Zelltyps durch einen magnetischen Durchflusszytometer vor dem Abtrennen der phagozytierten Zellen des ersten Zelltyps erfolgt. Das hat den Vorteil, dass im Anschluss eine genau definierte Probe der phagozytierten Zellen des ersten Zelltyps entnommen werden kann, insbesondere in Kombination mit einem Waschen ist diese Probe dann hervorragend für Sequenzierungs-Technologien verwendbar, welche eine genau definierte Probe benötigen.

In einer besonders vorteilhaften Ausführungsform ist vorgesehen, dass ein Lysieren der phagozytierten Zellen des ersten Zelltyps, nach dem Abtrennen und insbesondere nach dem Waschen der phagozytierten Zellen des ersten Zelltyps erfolgt und/oder, insbesondere anschließend, ein Amplifizieren der Nukleinsäuren der phagozytierten Zellen des ersten Zelltyps durchgeführt wird, insbesondere mittels einer Polymerase-Kettenreaktion. Dies bringt den Vorteil einer hochdefinierten Probe der Zellen des ersten Zelltyps, nämlich hoher Titer und aufgereinigte Nukleinsäuren, für eine Molekulardiagnostik. Diese hochdefinierte Probe wurde dabei erzeugt, ohne dass ein aufwendiges Lysieren von Zellen aller Zelltypen in der biologischen Probe erforderlich gewesen wäre. Diese genau definierte Probe kann auch sehr schnell, z.B. in weniger als einer Stunde, erzeugt werden. Das macht das Verfahren geeignet für Molekulardiagnostik in zeitkritischen Szenarien, zum Beispiel in einem Schockraum eines Krankenhauses. Gerade weil in diesem Verfahren auch eine geringe Konzentration von den Zellen des ersten Zelltyps gut nachgewiesen werden kann, eignet sich das Verfahren insbesondere zu einer schnellen Stratifizierung von Sepsispatienten. Durch das Bestimmen der genau definierten Probe, genauer das Bestimmen der Nukleinsäuren der Zellen des ersten Zelltyps, ist gleichzeitig auch die initiale biologische Probe bestimmt, in welcher ja dieselben Zellen des ersten Zelltyps mit den zugehörigen Nukleinsäuren enthalten sind. Somit ist es auch ermöglicht, die ursprüngliche biologische Probe nach der Analyse der genau definierten Probe zur Anreicherung der Nukleinsäure heranzuziehen.

Es kann in dem Verfahren entsprechend vorgesehen sein, dass die biologische Probe nach dem Abtrennen der phagozytierenden Zellen zum Anreichern der Nukleinsäuren der Zellen des ersten Zelltyps verwendet wird. Insbesondere erfolgt dies gemäß einer der geschilderten Verfahrensvarianten, wobei dann das Verfahren mit einem Großteil der biologischen Probe, insbesondere der gesamten biologischen Probe, unter Verwendung einer vorbestimmten Anzahl von Antikörpern und/oder Markern, die ausreichend ist, sämtliche phagozytierenden Zellen zu markieren, durchgeführt wird, um eine möglichst große Menge der Nukleinsäuren anzureichern. Das hat den Vorteil, dass einerseits für ein Anreichern einer größeren Menge der Nukleinsäuren dem Patienten keine neue biologische Probe abgenommen werden muss. Zudem ist bereits bekannt, ob bestimmte, möglicherweise teure und zeitaufwendige Verfahren zur Anreicherung, beispielsweise eine Aufkonzentrierung, überhaupt möglich oder erforderlich sind. Das hat eine Zeit- und Kostenersparnis zur Folge.

Die biologische Probe kann eine Urinprobe und/oder Lymphprobe und/oder Liquor, also insbesondere eine Probe einer Hirnoder Rückenmarksflüssigkeit, und/oder eine Blutprobe, insbesondere eine Vollblutprobe, umfassen. Das hat den Vorteil, dass die Probe direkt vom Patienten abgenommen werden kann, wie es für eine patientennahe Labordiagnostik wünschenswert ist. Blut, insbesondere Vollblut, bringt den Vorteil mit sich, dass bereits sehr früh erkannt werden kann, ob eine gefährliche Erkrankung oder Infektion vorliegt. Hier bringt die Verwendung von Vollblut den besonderen Vorteil mit sich, dass keine Verdünnung erforderlich ist, was nicht nur Zeit spart, sondern auch eine labortechnische Ausbildung des Bedienpersonals überflüssig macht.

Es ist insbesondere vorgesehen, dass es sich bei den ersten Zellen um Pathogene handelt, insbesondere um Pathogene, welche ein Exprimieren von CD64 in den phagozytierenden Zellen hervorrufen, und insbesondere es sich bei den Nukleinsäuren, die angereichert werden, um Anteile eines Genoms der Zellen des ersten Zelltyps oder gesamte Genome der Zellen des ersten Zelltyps handelt. Das hat den Vorteil, dass ein schnelles Verfahren zum Anreichern von Nukleinsäuren von Pathogenen zur Verfügung gestellt wird, was bei einem infizierten Patienten wünschenswert ist. Gerade bei einer Sepsis ist eine schnelle Identifizierung und somit auch Anreicherung hoch relevant, da die Mortalität von Sepsispatienten ohne frühzeitige Diagnostik schnell steigt. Eine schnelle Sepsiserkennung ermöglicht somit lebensrettende Sofortmaßnahmen. Gerade bei einer Sepsis sind jedoch Pathogene vorhanden, welche eine CD64-Exprimierung hervorrufen. Das Anreichern teilweiser oder gesamter Pathogengenome ist vorteilhaft, um das Pathogen zu identifizieren und eine Resistenztestung durchzuführen.

Es kann auch vorgesehen sein, dass es sich bei den phagozytierenden Zellen um Granulozyten oder Leukozyten, besonders vorteilhaft jedoch um Neutrophilen und/oder Monozyten handelt, wobei die Neutrophilen und/odere Monozyten dann insbesondere CD64 exprimierende Neutrophile und/oder Monozyten sind. Das hat den Vorteil, dass es sich bei den phagozytierenden Zellen um Zellen handelt, welche natürlicherweise im Blut vorhanden sind. Gerade Neutrophilen sind dafür bekannt, dass sie hocheffizient in kurzer Zeit, circa 30 Sekunden, opsonierte Pathogene umschließen können, vgl. hierzu J Cell Biol, 85, 42-59 (1980) und J. Leukoc. Biol. 90: 271-284 (2011). Die Verwendung von polymorphnuklearen Zellen, vorzugsweise CD64 exprimierenden Neutrophilen, bringt den Vorteil, dass ein hochspezifischer Test für eine Sepsisfrüherkennung möglich ist.

Weitere Merkmale ergeben sich aus der Beschreibung einer bevorzugten Ausführungsvariante gemäß den Figuren. Dabei zeigt
- FIG 1: schematisch eine beispielhafte Ausführungsform des erfindungsgemäßen Verfahrens; und
- FIG 2: schematisch eine weitere beispielhafte Ausführungsform des erfindungsgemäßen Verfahrens.

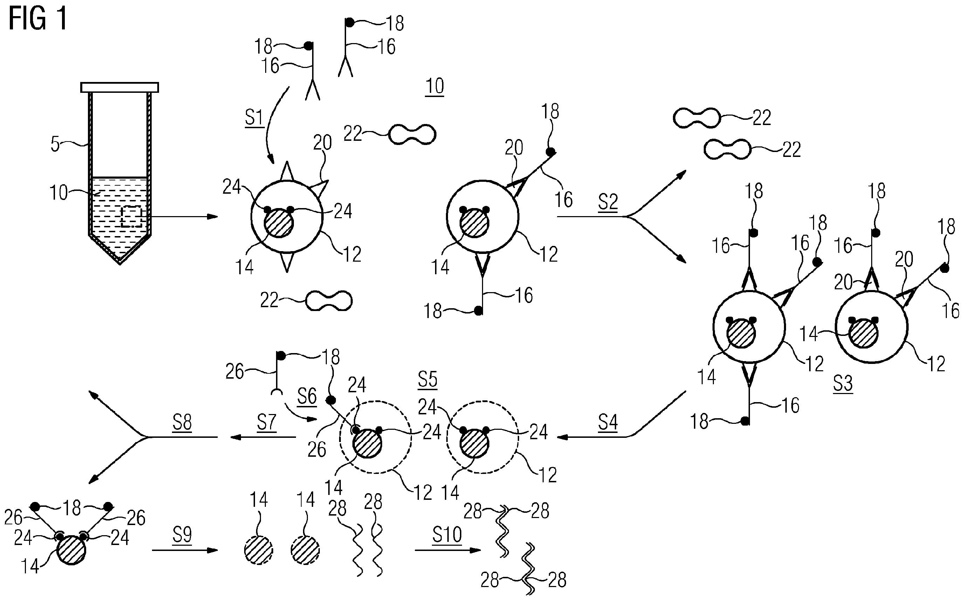

In dem in der FIG 1 dargestellten beispielhaften Verfahrensablauf wird ein sehr schneller Zellfunktionstest von beispielsweise CD64 exprimierenden Phagozyten, zum Beispiel Neutrophilen als phagozytierende Zellen 12, an einer sehr frischen unbehandelten biologischen Probe 10, welche im vorliegenden Beispiel weniger als eine Stunde vor Durchführen des Verfahrens einem Patienten entnommen wurde, verwendet. Dies erfolgt, um gleichzeitig eine Früherkennung beispielsweise einer Sepsis vorzunehmen, die beispielhaft getesteten Neutrophilen als standardisierte und angereicherte Probe für eine Molekulardiagnostik einzusetzen und in der biologischen Probe 10 möglicherweise vorhandene Inhibitoren für die Polymerase-Kettenreaktion wie Hämoglobin oder freie DNA zu eliminieren. Durch die Kombination von Nachweis und Anreicherung der beispielhaften Neutrophilen als phagozytierende Zellen 12 wird wertvolle Zeit bei der Sepsisdiagnostik eingespart. Weiterhin kann so einer Nukleinsäurediagnostik eine standardisierte angereicherte Probe ohne besagte Inhibitoren zur Verfügung gestellt werden. Dies ist vorteilhaft, da neuere und teure Next Generation Sequencing (NGS)-Technologien eine in Konzentration und Reinheit standardisierte Probe benötigen. Durch die Abklärung im präanalytischen Anreicherungsverfahren, ob eine Probe geeignet ist für eine NGS-Methode, können Kosten und Zeit in der Akutdiagnostik eingespart werden.

In dem in FIG 1 dargestellten Ausführungsbeispiel wird eine beispielsweise CD64 Exprimierung als Zellmembranprotein 20 von phagozytierenden Zellen 12, hier Neutrophilen, durch magnetische Durchflusszytrometrie in beispielsweise einer unverdünnten Vollblutprobe als biologische Probe 10, in der FIG 1 in einem Probengefäß 5 dargestellt, getestet. Dabei erfolgt zunächst ein Markieren (Verfahrensschritt S1) durch Antikörper 16 gegen vorliegend CD64 mit z.B. superparamagnetischen Markern 18 als spezifische Markierung. Die Antikörper 16 binden hier also lediglich an Zellmembranproteine 20 der phagozytierenden Zellen 12, nicht an restliche Probenbestandteile 22.

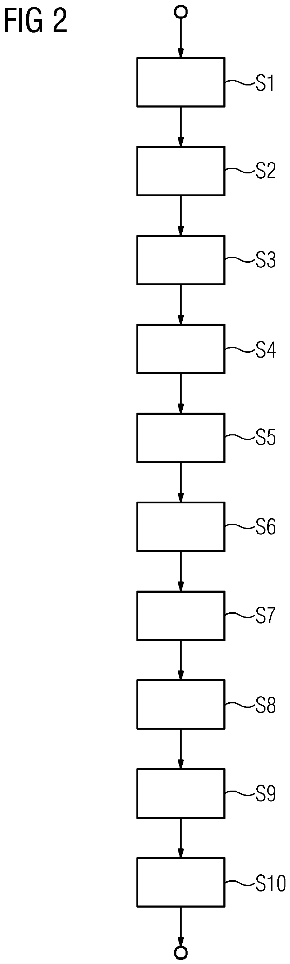

Es könnte hier nach dem Markieren auch ein Quantifizieren (Verfahrensschritt S2, FIG 2), beispielsweise über eine spezifische Detektion von z.B. CD64 exprimierenden Neutrophilen durch eine Messung von Volumen und Fließgeschwindigkeit der markierten Zellen und/oder durch eine magnetische Laufzeitmessung durch ein Durchflusszytometer erfolgen. Die Laufzeitmessung ermöglicht z.B. eine Korrelation zwischen phagozytierenden und aktivierten Neutrophilen. Eventuell in der biologischen Probe 10 vorhandene Monozyten exprimieren z.B. CD64 zeitverzögert und auf niedrigerem Niveau als hier die Neutrophilen. Nachdem Monozyten größer sind als Neutrophile und die Monozyten im Verhältnis zu Neutrophilen anteilig nur zu etwa 1 bis 10 Prozent im unverdünnten Vollblut vorkommen, kann so eine weitgehend spezifische Neutrophilendetektion erreicht werden. Durch den magnetosensitiven Nachweis der z.B. CD64 exprimierenden Neutrophilen kann also eine Zellkonzentration der Neutrophilen ermittelt werden und alle detektierten, z.B. CD64 exprimierenden Neutrophilen hinsichtlich ihrer Phagozytoseaktivität quantifiziert werden.

Es folgt hier ein Abtrennen (Verfahrensschritt S3), bei dem die angereicherten und quantifizierten Neutrophilen von den restlichen Probenbestandteilen 22, z.B. Erythrozyten, beispielsweise unmittelbar nach dem magnetosensitiven Nachweis, wenn ein solcher erfolgt ist, von der biologischen Probe 10 abgetrennt werden, beispielsweise in einer mikrofluidischen Kammer einer Zellsortiereinrichtung, durch welche das Blut als Volumenstrom hindurchfließt. Dieses Abtrennen kann in wenigen Minuten erfolgen. Nach Abschluss des Abtrennens kann zum Beispiel ein Waschen (Verfahrensschritt S4) der abgetrennten Zellen, z.B. Neutrophilen, von nicht markierten Blutkomponenten durchgeführt werden, beispielsweise durch eine dem Fachmann bekannte und für einen Waschschritt geeignete Pufferlösung in einem Sortiertopf der Zellsortiereinrichtung unter Anwesenheit eines Magnetfeldes. Die so angereicherten und gereinigten Zellen, hier CD64 exprimierende Neutrophilen, können im Anschluss für die Folgediagnostik oder für eine weitere Anreicherung aus der Zellsortiereinrichtung oder einer insbesondere zu der Zellsortiereinrichtung gehörigen Kartusche oder Cartridge entnommen werden. Da die Probe der z.B. CD64 exprimierenden Neutrophilen vorliegend sehr sauber und im Falle einer durchgeführten Quantifizierung auch genau bestimmt ist, reicht bereits geringer Anteil der biologischen Probe 10 von wenigen Mikrolitern für die weiteren Verfahrensschritte.

Mit dem Abtrennen (Verfahrensschritt S3) und Waschen (Verfahrensschritt S4) erreicht man, dass die Polymerase-Kettenreaktion störendes Hämoglobin, freie DNA und die DNA nicht markierter Neutrophile entfernt wurde, ohne dass es dabei erforderlich gewesen wäre, beispielsweise eine selektive Lyse von Erythrozyten oder andere aufwendige Aufreinigungsschritte durchzuführen. Damit kann im Vergleich zu kommerziellen Aufreinigungsverfahren eine Reduktion des die Polymerase-Kettenreaktion störenden Hintergrundes um bis zu 99 Prozent bezogen auf die Gesamtzellzahl im Blut erreicht werden.

Ein Anteil der aufgereinigten phagozytierenden Zellen 12, hier CD64 exprimierende Neutrophilen, in dem zu einem geringen Anteil auch z.B. CD64 exprimierende Monozyten enthalten sein können, wird nun beispielsweise in eine weitere, insbesondere zur ersten Zellsortiereinrichtung baugleiche Zellsortiereinrichtung gegeben. Dort findet dann in der hier beschriebenen Verfahrensvariante innerhalb von wenigen Minuten ein selektives Lysieren (Verfahrensschritt S5) der phagozytierenden Zellen 12, hier der Neutrophilen, beispielsweise durch einen pH-Wert von 11, statt. Die lysierten Zellen sind in der FIG 1 gestrichelt dargestellt. Hierauf folgt ein Markieren (Verfahrensschritt S6), in diesem Fall der phagozytierten Zellen 14, hier Pathogene, durch ein, hier pathogenunspezifisches, magnetisches Markieren mittels eines weiteren Liganden 26, mittels beispielsweise Binden eines Opsonins, z.B. Komplementfaktoren, beispielsweise kovalent gebunden auf einem superparamagnetischen Bead oder kovalent gebundene Immunoglobuline G oder Mischungen daraus an z.B. ein Oberflächenprotein 24 der phagozytierten Zelle 14. Hierauf erfolgt beispielsweise ein Erfassen (Verfahrensschritt S7) der markierten Zellen, hier der Pathogene, welche z.B. wieder magnetosensitiv, beispielsweise magnetoresistiv über einen magnetischen Durchflusszytometer, quantifiziert werden können. Auch findet hier nach dem Erfassen (Verfahrensschritt S7) ein Abtrennen (Verfahrensschritt S8) der zuvor phagozytierten Zellen 14, hier der Pathogene, in der weiteren Zellsortiereinrichtung mit Hilfe eines magnetischen Feldes statt. Das magnetische Feld kann aufgrund der magnetosensitiven Quantifizierung bereits von der für das Quantifizieren genutzten Vorrichtung vorhanden sein. Die zuvor phagozytierten Zellen 14, hier die Pathogene, können nun aus dem Sortiertopf des weiteren Zellsortiereinrichtung pipettiert werden. Für eine Molekulardiagnostik kann dann ein Lysieren (Verfahrensschritt S9) der zuvor phagozytierten Zellen 14 vorgesehen sein, auf welches ein Amplizieren (Verfahrensschritt S10) beispielsweise einer pathogenen Nukleinsäure 28 oder des pathogenen Genoms beispielsweise mittels einer Polymerase-Kettenreaktion erfolgen kann.

Damit wird erreicht, dass der Verdau der phagozytierten Zellen 14 gestoppt wird und die nicht aufgeschlossenen Zellen, hier Pathogene, von dem Genom und Granulae-Komponenten der phagozytierenden Zellen, in diesem Beispiel der Neutrophilen, selektiv gereinigt werden kann. Hier können beispielsweise die Membrankomponenten von den Phagozyten, also vorliegend den Neutrophilen und den wenigen in der Probe enthaltenen Monozyten, mit z.B. CD64 Label in situ durch Magnetophorese gefiltert werden. Dies erlaubt eine genau definierte Pathogenprobe aus Titer und reinem Pathogengenom für eine Molekulardiagnostik.

Gegebenenfalls kann bei dem Erfassen der markierten phagozytierten Zellen 14 (Verfahrensschritt S7) auch auf die magnetosensitive Messung verzichtet werden, und zum Beispiel ein immunomagnetisches Abtrennen der Pathogene, also allgemein der phagozytierten Zellen 14, sowie ein Waschschritt von dem Phagozytengenom durchgeführt werden. Bei einer positiven Sepsisfrüherkennung kann das im Zellsorter verbliebene Blut für eine Präanalytik zur Anreichung der pathogenen Genome herangezogen werden. Hierbei können die oben beschriebenen Arbeitsschritte des Erfassens (Verfahrensschritt S1), des Abtrennens (Verfahrensschritt S3), des Waschens (Verfahrensschritt S4), des selektiven Lysierens (Verfahrensschritt S5) sowie des Markierens der phagozytierten Zellen 14 (Verfahrensschritt S6), des Abtrennens der phagozytierten Zellen 14 (Verfahrensschritt S8), des Lysierens der phagozytierten Zellen 14 (Verfahrensschritt S9) und des Amplifizierens (Verfahrensschritt S10) durchgeführt werden, jedoch mit größeren Blutvolumina, um eine ausreichend hohe Menge an Pathogengenom anzureichern. Der Schritt des Erfassens der phagozytierenden Zellen 12 (Verfahrensschritt S2) und des Erfassens der phagozytierten Zellen (Verfahrensschritt S7), insbesondere eines Quantifizierens der markierten Pathogene, kann hier entfallen. Des Weiteren kann eine Anreicherung beispielsweise auch über einen Gradienten des oder eines weiteren Magnetfeldes in einer mikrofluidischen Strömung der Zellsortiereinrichtung erfolgen.

In dem in der FIG 2 dargestellten beispielhaften Verfahrensablauf wird ein sehr schneller Zellfunktionstest von CD64 exprimierenden Neutrophilen, an einer frischen biologischen Probe, welche z.B. weniger als eine Stunde vor Durchführen des Verfahrens einem Patienten entnommen wurde, verwendet. Dies erfolgt, um gleichzeitig eine Früherkennung einer Sepsis vorzunehmen, die getesteten Neutrophilen als standardisierte und angereicherte Probe für eine Molekulardiagnostik einzusetzen und in der Probe möglicherweise vorhandene Inhibitoren für die Polymerase-Kettenreaktion wie Hämoglobin oder freie DNA zu eliminieren. Durch die Kombination von Nachweis und Anreicherung der Neutrophilen wird wertvolle Zeit bei der Sepsisdiagnostik eingespart. Weiterhin kann so einer Nukleinsäurediagnostik eine standardisierte angereicherte Probe ohne besagte Inhibitoren zur Verfügung gestellt werden. Dies ist vorteilhaft, da neuere und teure Next Generation Sequencing (NGS)-Technologien eine in Konzentration und Reinheit standardisierte Probe benötigen. Durch die Abklärung im präanalytischen Anreicherungsverfahren, ob eine Probe geeignet ist für eine NGS-Methode, können Kosten und Zeit in der Akutdiagnostik eingespart werden.

In dem in FIG 2 dargestellten Ausführungsbeispiel wird eine CD64 Exprimierung von phagozytierenden Neutrophilen durch magnetische Durchflusszytrometrie im unverdünnten Vollblut getestet. Dabei erfolgt zunächst ein Markieren (Verfahrensschritt S1) durch CD64 Antikörper mit superparamagnetischen Markern als spezifische Markierung. Im gezeigten Beispiel erfolgt ein Quantifizieren (Verfahrensschritt S2) über eine spezifische Detektion von CD64 exprimierenden Neutrophilen durch eine Messung von Volumen und Fließgeschwindigkeit der markierten Zellen durch eine magnetische Laufzeitmessung. Diese Laufzeitmessung ermöglicht eine Korrelation zwischen phagozytierenden und aktivierten Neutrophilen. Eventuell in der biologischen Probe vorhandene Monozyten exprimieren CD64 zeitverzögert und auf niedrigerem Niveau. Nachdem Monozyten größer sind als Neutrophile und die Monozyten im Verhältnis zu Neutrophilen anteilig nur zu etwa 1 bis 10 Prozent im unverdünnten Vollblut vorkommen, kann so eine weitgehend spezifische Neutrophilendetektion erreicht werden. Durch den magnetosensitiven Nachweis der CD64 exprimierenden Neutrophilen kann also eine Zellkonzentration der Neutrophilen festgestellt werden und alle detektierten CD64 exprimierten Neutrophilen hinsichtlich ihrer Phagozytoseaktivität quantifiziert werden.

Auf das Quantifizieren (Verfahrensschritt S2) erfolgt hier ein Abtrennen oder Separieren (Verfahrensschritt S3), bei dem die angereicherten und quantifizierten Neutrophilen unmittelbar nach dem magnetosensitiven Nachweis, der magnetosensitiven Detektion, vom Volumenstrom des Blutes separiert werden, beispielsweise in einer Kammer der Mikrofluidik eines Zellsorters, durch welchen das Blut strömt. Diese Testung kann in wenigen Minuten erfolgen. Nach Abschluss der Testung kann zum Beispiel ein Waschen (Verfahrensschritt S4) der separierten Neutrophilen von nicht markierten Blutkomponenten durchgeführt werden, beispielsweise durch eine Pufferspülung in einem Sortertopf des Zellsorters unter Anwesenheit eines externen Magnetfeldes. Die angereicherten und gereinigten CD64 exprimierenden Neutrophilen können im Anschluss für die Folgediagnostik oder für eine weitere Anreicherung aus dem Zellsorter oder einer Cartridge entnommen werden. Da die Probe der CD64 exprimierenden Neutrophilen vorliegend sehr sauber und gut definiert ist, reicht bereits eine Probe von wenigen Mikrolitern für die weiteren Schritte.

Mit dem Abtrennen (Verfahrensschritt S3) und Waschen (Verfahrensschritt S4) erreicht man, dass die Polymerase-Kettenreaktion hemmendes Hämoglobin, freie DNA und die DNA nicht markierter Neutrophile entfernt wurde, ohne dass es dabei erforderlich gewesen wäre, beispielsweise eine selektive Lyse von Erythrozyten oder andere aufwendige Aufreinigungsschritte durchzuführen. Das ist im Vergleich zu kommerziellen Aufreinigungsverfahren eine Reduktion des die Polymerase-Kettenreaktion störenden Hintergrundes von 99 Prozent bezogen auf die Gesamtzellzahl im Blut.

Eine Probe der aufgereinigten CD64 exprimierenden Neutrophilen, in der zu einem geringen Anteil auch CD64 exprimierende Monozyten enthalten sein können, wird nun beispielsweise in einen weiteren Zellsorter gegeben. Dort findet dann in der hier beschriebenen Verfahrensvariante innerhalb von wenigen Minuten ein selektives Lysieren (Verfahrensschritt S5) der Neutrophilen, beispielsweise durch einen pH-Wert von 11, statt. Hierauf folgt ein Markieren (Verfahrensschritt S6), in diesem Fall der phagozytierenden Pathogene, durch eine, hier pathogenunspezifische, magnetische Markierung, beispielsweise Opsonine, Immunoglobolin G kovalent gebunden auf superparamagnetischen Beads oder Mischungen daraus. Hierauf erfolgt beispielsweise ein Erfassen (Verfahrensschritt S7) der markierten Pathogene, welche wieder magnetosensitiv, beispielsweise magnetoresistiv über einen magnetischen Durchflusszytometer, quantifiziert werden können. Auch findet hier nach dem Erfassen der Pathogene (Verfahrensschritt S7) ein Abtrennen der Pathogene (Verfahrensschritt S8) in dem weiteren Zellsorter mit Hilfe eines externen magnetischen Feldes statt. Das magnetische Feld ist aufgrund der magnetosensitiven Quantifizierung bereits vorhanden. Die zuvor phagozytierten Pathogene können nun aus dem Sortertopf des weiteren Zellsorters pipettiert werden. Für eine Molekulardiagnostik kann dann ein Lysieren der Pathogene (Verfahrensschritt S9) vorgesehen sein, auf welches ein Amplizieren (10) einer pathogenen Nukleinsäure oder des pathogenen Genoms beispielsweise mittels einer Polymerase-Kettenreaktion erfolgt.

Damit erreicht man, dass der Verdau der phagozytierten Pathogene gestoppt wird und die nicht aufgeschlossenen Pathogene von dem neutrophilen Genom und Granulae-Komponenten selektiv gereinigt werden kann. Hier können beispielsweise die Membrankomponenten von den Phagozyten, also den Neutrophilen und den wenigen in der Probe enthaltenen Monozyten, mit CD64 Label in situ durch Magnetophorese gefiltert werden. Dies erlaubt eine hochdefinierte Pathogenprobe aus Titer und reinem Pathogengenom für eine Molekulardiagnostik.

Gegebenenfalls kann bei dem Erfassen der Pathogene (Verfahrensschritt S7) auch auf die magnetosensitive Messung verzichtet werden, und zum Beispiel eine immunomagnetische Separation der Pathogene sowie ein Waschschritt von dem Pharozytengenom durchgeführt werden. Bei einer positiven Sepsisfrüherkennung kann das im Zellsorter verbliebene Blut für eine Präanalytik zur Anreichung der pathogenen Genome herangezogen werden. Hierbei können die oben beschriebenen Arbeitsschritte des Erfassens (Verfahrensschritt S1), des Abtrennens (Verfahrensschritt S3), des Waschens (Verfahrensschritt S4), des selektiven Lysierens (Verfahrensschritt S5) sowie des Markierens der Pathogene (Verfahrensschritt S6), des Abtrennens der Pathogene (Verfahrensschritt S8), des Lysierens der Pathogene (Verfahrensschritt S9) und des Amplifizierens (Verfahrensschritt S10) durchgeführt werden, jedoch mit größeren Blutvolumina, um eine ausreichend hohe Menge an Pathogengenom anzureichern. Der Schritt des Erfassens der Neutrophilen (Verfahrensschritt S2) und des Erfassens der Pathogene(Verfahrensschritt S7), insbesondere eines Quantifizierens der markierten Pathogene, kann hier entfallen. Des Weiteren kann eine Anreicherung auch über einen Gradienten des externen Magnetfeldes in einer mikrofluidischen Strömung des Zellsorters erfolgen.

In einem weiteren, nicht dargestellten Ausführungsbeispiel eines Präanalyseverfahrens werden beispielsweise ca. 60 Minuten für die Vorbereitung der Probe entsprechend den Verfahrensschritten S1 bis S8 aufgewendet. Das darauf folgende Isolieren der Nukleinsäure, beispielsweise einer DNA eines Pathogens, entsprechend dem Verfahrensschritt S9 erfordert rund weitere 60 Minuten. Die Polymerase-Kettenreaktion (PCR) gemäß Verfahrensschritt S10 kann dann in 30 Minuten durchgeführt werden. Es sind dann noch rund 30 Minuten für das Ermitteln der Produkte der Polymerase-Kettenreaktion aufzuwenden, bevor nach ca. 4 Stunden nach Beginn des Präanalyseverfahrens erste klinische Information zur Verfügung steht. So kann hier beispielsweise bereits festgestellt werden, ob der Patient keine Infektion hat, von grampositiven oder gramnegativen Bakterien infiziert oder von einem Pilz befallen ist, oder sogar mehrfach infiziert oder befallen ist. Das weitere Aufbereiten bis zur genauen Identifikation des Befalls und einer entsprechenden Dokumentation inklusive einer Sequenzierung kann dann beispielsweise noch weitere 180 Minuten dauern.

In einem weiteren nicht dargestellten Ausführungsbeispiel werden in einem ersten Test- und Abtrenn-Schritt magnetisch markierte phagozytierende Zellen, beispielsweise polymorphnukleäre Zellen und Monozyten, nach dem Überströmen eines Riesenmagnetowiderstandssensors in einer Kartusche abgetrennt. Damit kann ein für weitere Verfahrensschritte benötigtes Volumen der abgetrennten phagozytierenden Zellen auf rund 20µl reduziert werden. Bei eine positiven Ergebnis, welches beispielsweise auf ein frühes Stadium einer Sepsis, z.B. mit einer Konzentration z.B. eines Pathogens mit weniger als 500CFU/ml in der Probe, hinweist, können die abgetrennten phagozytierenden Zellen beispielsweise für weitere molekulardiagnostische Maßnahmen verwendet werden. Der Test- und Abtrenn-Schritt erfordert hier rund 11 Minuten.

In einem zweiten Wasch-Schritt werden in diesem Beispiel dann Blutplasma, rote Blutkörperchen, nicht-markierte weiße Blutkörperchen und freie DNA aus den bereits abgetrennten phagozytierende Zellen ausgewaschen und entfernt. Vor allem wird hier der Polymerase-Kettenreaktion-Inhibitor Hämoglobin entfernt, welcher ca. 35% des gesamten Blutvolumens ausmacht. Dafür wird hier eine Spritze mit einer Waschlösung an die Kartusche angeschlossen. Die Waschlösung kann dann z.B. durch die Kartusche hindurch an den abgetrennten phagozytierenden Zellen vorbei gepresst werden. Von der Waschlösung werden dann Blutplasma, rote Blutkörperchen, nicht-markierte weiße Blutkörperchen und freie DNA mitgerissen. Die markierten phagozytierenden Zellen werden dabei beispielsweise durch ein Magnetfeld festgehalten, sodass sie in der Kartusche vorliegend an einem vorbestimmten Ort verbleiben. Der Wasch-Schritt dauert hier etwa 3 Minuten.

In einem Transfer- und Sortier-Schritt werden die abgetrennten phagozytierenden Zellen zunächst von dem vorbestimmten Ort in der Kartusche in eine weitere Kartusche transportiert. Dort findet ein selektives Lysieren der phagozytierenden Zellen, also hier der polymorphnukleären Zellen und Monozyten, statt, z.B. bei einem pH-Wert von 11. Die Membrankomponenten der ja magnetisch markierten phagozytierenden Zellen werden in diesem Beispiel dann magnetisch abgetrennt. In der weiteren Kartusche erfolgt dann ein Markieren, hier ein unspezifisches Markieren von Bakterien durch Opsonin-Kombinationen, die jegliche grampositiven und gramnegativen Bakterien markieren. Die markierten Bakterien werden dann abgetrennt und eventuell noch vorhandenes Genom der phagozytierenden Zellen analog zu obigem Wasch-Schritt ausgewaschen. Hier kann auch wieder ein Riesenmagnetowiderstandssensor verwendet werden, um die Bakterien zu quantifizieren. Für den Transfer- und Sortier-Schritt werden ungefähr 11 Minuten benötigt.

Insgesamt kann so im vorliegenden Beispiel innerhalb von 25 Minuten nach einem Markieren von phagozytierenden Zellen ein Transfer zu einem Sequenzierer erfolgen. Dort kann eine kleine, wenige µl umfassende Probe der in diesem Beispiel quantifizierten Bakterien lysiert werden, woraufhin eine Amplifizierung und Sequenzierung erfolgen kann. Alternativ kann das Lysieren der Bakterien auch noch in der weiteren Kartusche erfolgen.

## Patentansprüche

1. Verfahren für die Molekulardiagnostik, zum Anreichern von Nukleinsäuren phagozytierter Zellen eines ersten Zelltyps aus einer zuvor entnommenen biologischen Probe eines Patienten, wobei die biologische Probe Zellen eines weiteren, phagozytierenden Zelltyps, die jeweils eine Zelle des ersten Zelltyps phagozytieren, umfasst,
bei dem
- ein Markieren (1) von zumindest einem Anteil der phagozytierenden Zellen der biologischen Probe durch für die phagozytierenden Zellen spezifische Antikörper erfolgt, wobei die Antikörper jeweils an einen magnetischen Marker gekoppelt sind und wobei die Antikörper spezifisch für Zellmembranproteine eines vorbestimmten Zellmembranproteintyps, welche von den phagozytierenden Zellen nur im Falle einer erfolgten Phagozytose exprimiert werden, sind, gefolgt von
- einem Abtrennen (3) der markierten phagozytierenden Zellen aus der biologischen Probe mittels eines durch eine Zelltrennungsvorrichtung angelegten Magnetfeldes, wodurch ein Anreichern der markierten phagozytierenden Zellen und damit ein Anreichern der Nukleinsäuren der phagozytierten Zellen erfolgt.

2. Verfahren nach Anspruch 1, **dadurch gekennzeichnet, dass**
das Markieren mit jeweils an einen paramagnetischen Marker gekoppelten Antikörpern erfolgt und vor dem Abtrennen (3) ein Erfassen (2) der markierten phagozytierenden Zellen durch ein magnetisches Durchflußzytometer erfolgt, wobei das Erfassen (2) insbesondere ein Quantifizieren umfasst, und das Quantifizieren insbesondere ein Bestimmen einer Anzahl der markierten phagozytierenden Zellen, insbesondere pro durch das Durchflusszytometer geströmten Probenvolumen, und/oder ein Bestimmen eines Durchmessers und/oder eines Volumens und/oder einer Fließgeschwindigkeit jeweiliger markierter phagozytierender Zellen und/oder einer Durchmesserverteilung der markierten phagozytierenden Zellen umfasst.

3. Verfahren nach einem der vorhergehenden Ansprüche, **dadurch gekennzeichnet,**
**dass**
- das Abtrennen (3) der markierten phagozytierenden Zellen von einem Volumenstrom der zuvor entnommenen biologischen Probe in einer Zellsortiereinrichtung mittels des angelegten Magnetfeldes in einen Sortiertopf der Zellsortiereinrichtung erfolgt und
- ein Reinigen (4) der separierten phagozytierenden Zellen in dem Sortiertopf mittels einer durch die Zellsortiereinrichtung strömenden Pufferlösung auf das Abtrennen folgt, insbesondere unter Vorliegen des angelegten Magnetfeldes.

4. Verfahren nach einem der vorhergehenden Ansprüche, **dadurch gekennzeichnet,**
**dass**
- ein selektives Lysieren (5) eines vorbestimmten Anteils der abgetrennten phagozytierenden Zellen stattfindet, sowie danach
- ein Markieren (6) der von den lysierten Zellen phagozytierten ersten Zellen mit magnetischen, insbesondere paramagnetischen, Markern, gefolgt von
- einem Abtrennen (8) der markierten phagozytierten ersten Zellen in einer weiteren Zellsortiereinrichtung in einen Sortiertopf der weiteren Zellsortiereinrichtung mittels eines weiteren oder desselben angelegten Magnetfeldes.

5. Verfahren nach Anspruch 4, **dadurch gekennzeichnet, dass** ein Waschen der phagozytierten ersten Zellen in dem Sortiertopf mittels einer durch den Zellsortierer strömenden Pufferlösung auf das Abtrennen (8) der markierten phagozytierten Zellen folgt, insbesondere während des Bereitstellens des Magnetfeldes.

6. Verfahren nach Anspruch 4 oder 5, **dadurch gekennzeichnet, dass** ein Erfassen (7), insbesondere ein Quantifizieren, der phagozytierten ersten Zellen durch ein magnetisches Durchflußzytometer vor dem Abtrennen (8) der phagozytierten ersten Zellen erfolgt.

7. Verfahren nach Anspruch 4 oder 5 oder 6, **dadurch gekennzeichnet, dass**
- ein Lysieren (9) der abgetrennten phagozytierten ersten Zellen, insbesondere nach dem Waschen der phagozytierten ersten Zellen, erfolgt, und/oder
- ein Amplifizieren (10) der Nukleinsäuren der phagozytierten ersten Zellen durchgeführt wird, insbesondere mittels der Polymerase-Kettenreaktion.

8. Verfahren nach einem der vorhergehenden Ansprüche, **dadurch gekennzeichnet, dass** die zuvor entnommene biologische Probe eine Urinprobe und/oder Liquor und/oder Lymphprobe und/oder Blutprobe, insbesondere eine Vollblutprobe umfasst.

9. Verfahren nach einem der vorhergehenden Ansprüche, **dadurch gekennzeichnet, dass**
es sich bei den ersten Zellen um Pathogene handelt, insbesondere um Pathogene, welche ein Exprimieren von CD64 in den phagozytierenden Zellen hervorrufen, und insbesondere es sich bei den Nukleinsäuren um Genome der ersten Zellen handelt.

10. Verfahren nach einem der vorhergehenden Ansprüche, **dadurch gekennzeichnet, dass**
es sich bei den phagozytierenden Zellen um Granulozyten oder Leukozyten oder Monozyten, besonders vorteilhaft um Neutrophilen handelt, welche insbesondere CD64 exprimierende Neutrophilen sind.

## Claims

1. A method for molecular diagnostics for enriching nucleic acids of phagocytized cells of a first cell type from a previously taken biological sample of a patient, wherein the biological sample comprises cells of a further phagocytizing cell type each phagocytizing a cell of the first cell type,
in which
- a marking (1) of at least a proportion of the phagocytizing cells of the biological sample is performed by antibodies which are specific for the phagocytizing cells, wherein the antibodies are each coupled to a magnetic marker and wherein the antibodies are specific for cell membrane proteins of a predetermined cell membrane protein type which are expressed by the phagocytizing cells only if phagocytosis has taken place, followed by
- a separation (3) of the marked phagocytizing cells from the biological sample by means of a magnetic field applied by a cell separation device, whereby an enrichment of the marked phagocytizing cells and thus an enrichment of the nucleic acids of the phagocytized cells takes place.

2. The method according to claim 1, **characterized in that**
the marking is carried out with antibodies coupled in each case to a paramagnetic marker and, prior to separation (3), a detection (2) of the marked phagocytizing cells is carried out by a magnetic flow cytometer, the detection (2) comprising in particular a quantification, and the quantification comprising in particular determining a number of the marked phagocytizing cells, in particular per sample volume that has flowed through the flow cytometer, and/or determining a diameter and/or a volume and/or a flow velocity of respective marked phagocytizing cells and/or a diameter distribution of the marked phagocytizing cells.

3. The method according to any of the preceding claims, **characterized in that**
- the separation (3) of the marked phagocytizing cells from a volume flow of the previously taken biological sample is carried out in a cell sorting device by means of the applied magnetic field into a sorting pot of the cell sorting device, and
- a cleaning (4) of the separated phagocytizing cells in the sorting pot by means of a buffer solution flowing through the cell sorting device is carried out after the separation, in particular in the presence of the applied magnetic field.

4. The method according to any of the preceding claims, **characterized in that**
- a selective lysis (5) of a predetermined proportion of the separated phagocytizing cells takes place, and thereafter
- a marking (6) of the first cells, phagocytized by the lysed cells, with magnetic markers, in particular paramagnetic markers, followed by
- a separation (8) of the marked phagocytized first cells in a further cell sorting device into a sorting pot of the further cell sorting device by means of a further magnetic field or the same magnetic field applied.

5. The method according to claim 4, **characterized in that**
a washing of the phagocytized first cells in the sorting pot by means of a buffer solution flowing through the cell sorter is carried out after the separation (8) of the marked phagocytized cells, in particular during provision of the magnetic field.

6. The method according to claim 4 or 5, **characterized in that**
a detection (7), in particular a quantification, of the phagocytized first cells is carried out by a magnetic flow cytometer prior to the separation (8) of the phagocytized first cells.

7. The method according to claim 4 or 5 or 6,
**characterized in that**
- a lysing (9) of the separated phagocytized first cells takes place in particular after the washing of the phagocytized first cells, and/or
- an amplification (10) of the nucleic acids of the phagocytized first cells is carried out in particular by means of the polymerase chain reaction.

8. The method according to any of the preceding claims, **characterized in that**
the biological sample previously taken comprises a urine sample and/or cerebrospinal fluid and/or a lymph sample and/or a blood sample, in particular a whole blood sample.

9. The method according to any of the preceding claims, **characterized in that**
the first cells are pathogens, in particular pathogens causing an expression of CD64 in phagocytizing cells, and the nucleic acids are in particular genomes of the first cells.

10. The method according to any of the preceding claims, **characterized in that**
the phagocytizing cells are granulocytes or leukocytes or monocytes, especially advantageous are neutrophils which are in particular neutrophils expressing CD64.

## Revendications

1. Procédé pour diagnostics moléculaires, pour enrichir des acides nucléiques de cellules phagocytées d'un premier type de cellules à partir d'un échantillon biologique d'un patient préalablement prélevé, l'échantillon biologique comprenant des cellules d'un autre type de cellules phagocytaires phagocytant respectivement une cellule du premier type de cellules ;
dans lequel :
- un marquage (1) d'au moins une partie des cellules phagocytaires de l'échantillon biologique se produit au travers d'anticorps spécifiques aux cellules phagocytaires, les anticorps étant respectivement couplés à un marqueur magnétique et les anticorps étant spécifiquement exprimés pour les protéines de membrane cellulaire d'un type de protéines de membrane cellulaire prédéfini exprimé par les cellules phagocytaires en cas seulement de phagocytose effective ; suivi de
- une séparation (3) des cellules phagocytaires marquées provenant de l'échantillon biologique à l'aide d'un champ magnétique appliqué par un dispositif de séparation de cellules, entraînant un enrichissement des cellules phagocytaires marquées et ainsi un enrichissement des acides nucléiques des cellules phagocytées.

2. Procédé selon la revendication 1, **caractérisé en ce que** le marquage est respectivement réalisé avec des anticorps couplés à un marqueur paramagnétique et qu'avant la séparation (3), une détection (2) des cellules phagocytaires marquées s'effectue par le biais d'un cytomètre de débit magnétique, la détection (2) comprenant notamment une quantification et la quantification comprenant notamment une détermination d'un nombre de cellules phagocytaires marquées, notamment par volume d'échantillon traversant le cytomètre de débit et/ou une détermination d'un diamètre et/ou d'un volume et/ou d'une vitesse d'écoulement des cellules phagocytaires marquées respectives et/ou d'une répartition par diamètre des cellules phagocytaires marquées.

3. Procédé selon l'une quelconque des revendications précédentes, **caractérisé en ce que** :
- la séparation (3) des cellules phagocytaires marquées par rapport à un débit volumique de l'échantillon biologique préalablement prélevé s'effectue dans un dispositif de tri de cellules par application d'un champ magnétique dans un bac de tri du dispositif de tri de cellules ; et
- un nettoyage (4) des cellules phagocytaires séparées dans le bac de tri au moyen d'une solution tampon s'écoulant à travers le dispositif de tri de cellules suit la séparation, notamment en présence du champ magnétique appliqué.

4. Procédé selon l'une quelconque des revendications précédentes, **caractérisé en ce que** :
- une lyse sélective (5) d'une partie prédéterminée des cellules phagocytaires séparées a lieu, ainsi qu'ensuite :
- un marquage (6) des premières cellules phagocytées par les cellules lysées à l'aide de marqueurs magnétiques, notamment paramagnétiques ; suivi de
- une séparation (8) des premières cellules phagocytées marquées dans un autre dispositif de tri de cellules dans un bac de tri de l'autre dispositif de tri de cellules par application d'un autre champ magnétique ou du même champ magnétique.

5. Procédé selon la revendication 4, **caractérisé en ce qu'**un lavage des premières cellules phagocytées dans le bac de tri au moyen d'une solution tampon s'écoulant à travers le dispositif de tri de cellules suit la séparation (8) des cellules phagocytées marquées, notamment pendant la mise à disposition du champ magnétique.

6. Procédé selon la revendication 4 ou 5, **caractérisé en ce qu'**une détection (7), notamment une quantification des premières cellules phagocytées, est effectuée par le biais d'un cytomètre de débit magnétique avant la séparation (8) des premières cellules phagocytées.

7. Procédé selon la revendication 4 ou 5 ou 6, **caractérisé en ce que** :
- une lyse (9) des premières cellules phagocytées séparées est réalisée, notamment après lavage des premières cellules phagocytées ; et/ou
- une amplification (10) des acides nucléiques des premières cellules phagocytées est réalisée, notamment grâce à la réaction en chaîne des polymérases.

8. Procédé selon l'une quelconque des revendications précédentes, **caractérisé en ce que** l'échantillon biologique préalablement prélevé comprend un échantillon d'urine et/ou un liquide corporel et/ou un échantillon de lymphe et/ou un échantillon de sang, notamment un échantillon de sang total.

9. Procédé selon l'une quelconque des revendications précédentes, **caractérisé en ce que** les premières cellules sont des pathogènes, notamment des pathogènes induisant une expression de CD64 dans les cellules phagocytaires, et notamment, dans les acides nucléiques, des génomes des premières cellules.

10. Procédé selon l'une quelconque des revendications précédentes, **caractérisé en ce que** les cellules phagocytaires sont des granulocytes ou des leucocytes ou des monocytes, de façon particulièrement avantageuse des neutrophiles, notamment des neutrophiles exprimant CD64.
